# EUROPEAN PATENT APPLICATION

(11) **EP 4 410 175 A1**
(43) Date of publication of application: **07.08.2024**
(21) Application number: 22875824.9
(22) Date of filing: 14.09.2022
(51) Int. Cl.: A61B 1/00, A61B 1/05

(54) **OPTICAL TRANSMITTER MODULE AND ENDOSCOPE**

(30) Priority: 01.10.2021 JP 2021162835
(71) Applicant: HOYA CORPORATION, Shinjuku-ku Tokyo 160-8347 (JP)
(72) Inventor: KOMATSU Masahiro, Tokyo 160-8347 (JP)
(74) Representative: Schaumburg und Partner Patentanwälte mbB
(86) International application number: PCT/JP2022/034297
(87) International publication number: WO 2023/053953

(57) **Abstract**

Provided are an optical transmitter module (30) and an endoscope (11) that prevent damage to an optical fiber (34) .

The optical transmitter module (30) includes an image sensor (31), a laser element (32), a laser driver circuit (33), an optical fiber (34), and a layered substrate (37). The optical fiber (34) is connected to the laser element (32), and at least a part of an outer periphery of the optical fiber (34) is covered with a protective member (38, 44). The endoscope (11) includes the optical transmitter module (30) described above.

## Description

### Technical Field

The present invention relates to an optical transmitter module and an endoscope.

### Background Art

An endoscope in which an image sensor is built in a distal end of an insertion portion is known. In order to enable clear image observation, an image sensor having a large number of pixels is used. In a case where an image sensor having a large number of pixels is used in the endoscope, in order to transmit a signal between the image sensor and a signal processing device at a high speed, an optical transmitter module is incorporated in the endoscope, and an optical fiber is used for signal transmission.

Examples of an optical transmitter module using an optical fiber are described in Patent Literature 1.

### Citation List

### Patent Literature

Patent Literature 1: JP 5919573 B1

### Summary of Invention

### Technical Problem

However, when the conventional optical transmitter module is used in the endoscope, there is the problem that the optical fiber may be damaged by the bending operation of the endoscope. More specifically, the optical fiber may be crushed or broken.

Patent Literature 1 describes a technique for increasing an optical coupling efficiency between a light emitting element and an optical fiber, but such a technique alone cannot prevent damage to the optical fiber.

The present invention has been made to solve such a problem, and aims to provide an optical transmitter module and an endoscope that prevent damage to the optical fiber.

### Solution to Problem

An optical transmitter module according to the present invention includes:
an image sensor, a laser element, a laser driver circuit, an optical fiber, and a layered substrate, in which
the optical fiber is connected to the laser element, and
at least a part of an outer periphery of the optical fiber is covered with a protective member.

An endoscope according to the present invention includes the optical transmitter module described above.

The present description includes the disclosure of Japanese Patent Application No. 2021-162835 on which priority of the present application is based.

### Advantageous Effects of Invention

According to an optical transmitter module and an endoscope according to the present invention, damage to the optical fiber can be prevented.

### Brief Description of Drawings

Fig. 1 is a diagram illustrating a configuration example of an endoscope system according to a first embodiment.
Fig. 2 is a diagram illustrating a configuration example of a distal end of the endoscope of Fig. 1.
Fig. 3 is a diagram illustrating a configuration example of an optical transmitter module of Fig. 2.
Fig. 4 is another diagram illustrating a configuration example of the optical transmitter module of Fig. 2.
Fig. 5 is a diagram illustrating an example of an axial position of the coil of Fig. 3.
Fig. 6 is a diagram illustrating a configuration example of a first cap member of Figs. 3 and 4.
Fig. 7 is a diagram illustrating an example of sealing with a molding agent.
Fig. 8 is a diagram illustrating a configuration example of an optical fiber and a tube according to a second embodiment.

### Description of Embodiments

Hereinafter, embodiments of the present invention will be described with reference to the accompanying drawings.

### First Embodiment

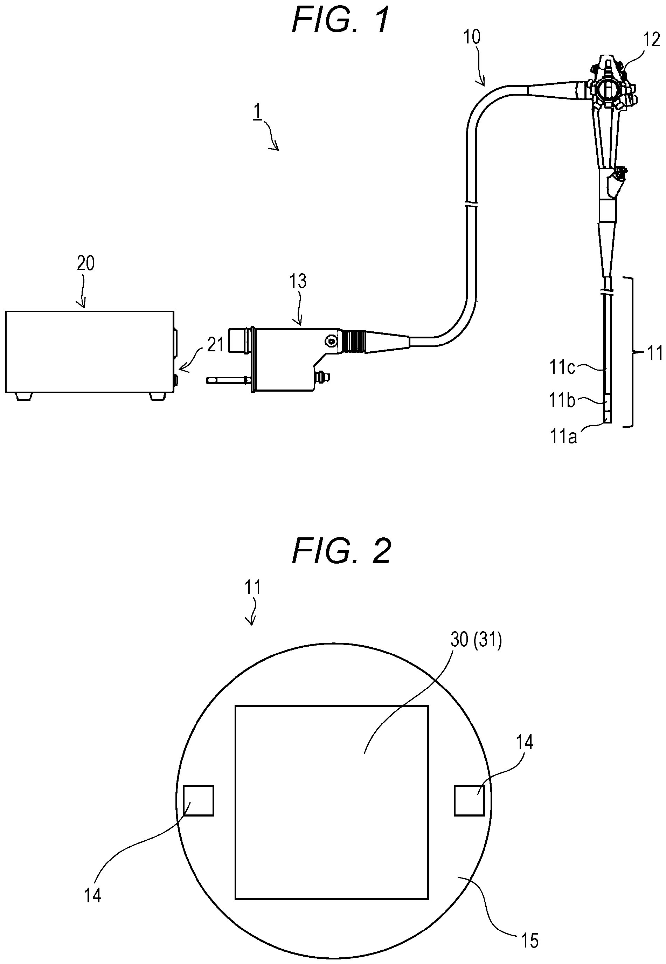

Fig. 1 is a diagram illustrating a configuration example of an endoscope system 1 according to the present embodiment. The endoscope system 1 may be what is called an electronic endoscope. As illustrated in Fig. 1, the endoscope system 1 is, for example, a dedicated system for medical use and includes an endoscope unit 10 and a processor 20.

As illustrated in Fig. 1, the endoscope unit 10 includes an endoscope 11 and an operation unit 12. The endoscope 11 includes, in order from the distal end, a hard portion 11a that is not deformed, a bend portion 11b that can actively bend in response to an operation, and a flexible portion 11c that can be passively deformed. A user of the endoscope system 1 operates the operation unit 12 to control a movement of the endoscope 11. For example, the bend portion 11b is bent depending on an operation of the operation unit 12. The bend portion 11b can be realized by using a known mechanism incorporated in a typical electronic scope, and is configured to bend the bend portion 11b by, for example, pulling an operation wire in conjunction with rotational operation of a knob included in the operation unit 12.

The endoscope 11 can insert a portion including the hard portion 11a into any body cavity in a living body, for example, into a bronchus, a biliary tract, a pancreas, a hepatic duct region, a urinary organ region, or the like.

The processor 20 integrally includes: a signal processing device that processes an image signal from the endoscope unit 10; and a light source device that applies light via the endoscope unit 10 to a body cavity to which no natural light can reach. Note that, in another embodiment, the signal processing device and the light source device may be configured separately.

A proximal end of the endoscope unit 10 is provided with a connector unit 13, and the processor 20 is provided with a connector unit 21. The connector unit 13 and the connector unit 21 each have a coupling structure corresponding to each other. Coupling of these structures electrically and optically connects the endoscope unit 10 and the processor 20.

The processor 20 functions as a controller that controls the entire endoscope system 1 and includes, for example, a computer provided with an arithmetic unit and a storage unit. Other functions and configurations of the endoscope unit 10 and the processor 20 (for example, the function of acquiring an image inside a body cavity or the like) can be appropriately designed by those skilled in the art based on known techniques. For example, the processor 20 performs various calculations on the basis of specific information of the endoscope unit 10 and generates a control signal. Furthermore, the processor 20 uses the generated control signal to control operations and timings of various circuits in the processor 20, thereby causing the endoscope unit 10 to move appropriately.

Note that, assuming that the endoscope unit 10 is connected to an appropriate processor other than the processor 20, the endoscope unit 10 may be provided independently.

Fig. 2 illustrates a configuration example of a distal end of the endoscope 11. Fig. 2 is a view (top view) of the distal end of the endoscope 11 as viewed from the distal end side in the axial direction. Note that, in the present description, the "axial direction" and the "radial direction" mean the axial direction and the radial direction of the endoscope 11, respectively. The endoscope 11 includes an optical transmitter module 30.

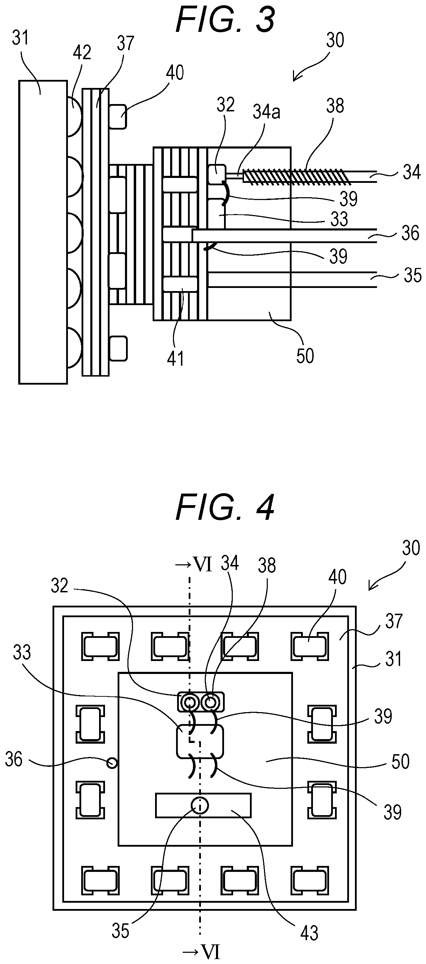

The configuration of the optical transmitter module 30 will be described later with reference to Figs. 3 and 4, for example. In Fig. 2, however, only an image sensor 31 of the optical transmitter module 30 appears. The optical transmitter module 30 is fixed in the endoscope 11 using a fixing member (for example, a rod 15). The structure of the fixing member is not limited to that shown in Fig. 2, and those skilled in the art can appropriately design the fixing member.

The endoscope 11 includes a light irradiation means 14. The light irradiation means 14 is provided at or near the distal end of the endoscope 11. The light irradiation means 14 is, for example, a ride guide, and more specifically, can be configured using an optical fiber.

Although not illustrated in Fig. 2, the endoscope 11 may further include other configurations included in a known endoscope. For example, the endoscope 11 may include a forceps channel, an air pipe, a water pipe, a water jet pipe, and the like. In that case, the distal end of the endoscope 11 illustrated in Fig. 2 may be provided with an opening or the like corresponding thereto.

Fig. 3 and Fig. 4 illustrate a configuration example of the optical transmitter module 30. Fig. 3 is a view (side view) of the optical transmitter module 30 as viewed from the radial direction, and Fig. 4 is a view (bottom view) of the optical transmitter module 30 as viewed from the rear end side in the axial direction.

The optical transmitter module 30 includes the image sensor 31 described above, a laser element 32, a laser driver circuit 33, at least one optical fiber 34 (two in the present embodiment), a heat dissipation wiring 35, at least one electric signal wiring 36, and a layered substrate 37. The image sensor 31, the laser element 32, the laser driver circuit 33, the optical fiber 34, the heat dissipation wiring 35, and the electric signal wiring 36 are mounted on the layered substrate 37.

The image sensor 31 is connected to the layered substrate 37 via a solder 42. The laser element 32 and the laser driver circuit 33 are mounted on the layered substrate 37 by die bonding and wire bonding, and in particular connected to the layered substrate 37 via wires 39. The laser element 32 and the laser driver circuit 33 constitute an electrical-optical converter that converts an electric signal output from the image sensor 31 into an optical signal.

The optical fiber 34 is connected to the laser element 32 and conveys the laser output from the laser element 32. The heat dissipation wiring 35 is connected to a ground potential pad 43 and discharges heat of the optical transmitter module 30 (for example, heat generated by the image sensor 31) via the ground potential pad 43.

A cable wiring pad 41 is provided on a side surface of the layered substrate 37 (that is, a surface parallel to the axis of the endoscope 11). Although three cable wiring pads 41 are illustrated in the example of Fig. 3, the number of the cable wiring pads 41 may be one or more. The electric signal wiring 36 is connected to the layered substrate 37 via the cable wiring pad 41. For convenience of illustration, only one electric signal wiring 36 is illustrated in the examples of Figs. 3 and 4, but the number of electric signal wiring 36 may be two or more, and for example, each of the cable wiring pads 41 may be connected to separated one electric signal wiring 36.

A passive component 40 (for example, a capacitor, a resistor, and the like) may be connected to the layered substrate 37. The passive component 40 may be connected to the electric signal wiring 36 via the layered substrate 37.

As described above, each component can be compactly arranged by a mounting style using the layered substrate 37, and the entire optical transmitter module 30 can be downsized.

A coil 38 as a protective member is wound around the optical fiber 34, and the outer periphery of the optical fiber 34 is covered with the coil 38. The coil 38 is made of, for example, metal and can have a structure having sufficient strength to prevent damage to the optical fiber 34. When the optical transmitter module 30 includes a plurality of optical fibers 34 as in the present embodiment, the single coil 38 may cover the outer periphery of the plurality of optical fibers 34, or each of a plurality of separate coils 38 may cover the outer periphery of one optical fiber 34.

Since the optical fiber 34 is covered with the coil 38, it is possible, when the endoscope performs the bending operation, to reduce the possibility that the optical fiber is damaged (for example, it is crushed or folded) at least in the covered portion. For example, the coil 38 can relieve a radial pressure applied to the optical fiber 34 in the endoscope 11, which can prevent the optical fiber 34 from being crushed.

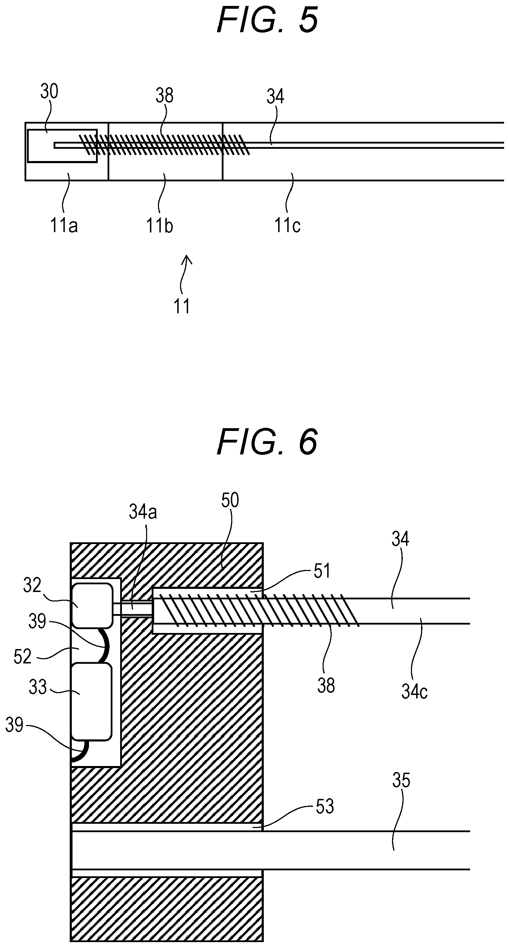

Fig. 5 illustrates an example of an axial position of the coil 38. In the present embodiment, the coil 38 covers the outer periphery of the optical fiber 34 at least over an entire length of the bend portion 11b (that is, a portion that can actively bend in response to an operation of the user) of the endoscope 11. In this way, a bending force that is applied to the optical fiber 34 as the endoscope 11 actively bend can be dispersed in a longer range, which can prevent the optical fiber 34 from being broken.

As described above, in the present embodiment, the coil 38 does not cover the outer periphery over an entire length of the optical fiber 34, but covers only a part of the outer periphery. In particular, a portion of the hard portion 11a where the core 34a of the optical fiber 34 is exposed (Fig. 3, that is, a connection portion with the laser element 32) is not covered, and a portion of the flexible portion 11c on the rear end side (processor 20 side) is also not covered.

The optical transmitter module 30 includes a first cap member 50 made of glass. As illustrated in Figs. 3 and 4, the first cap member 50 is disposed on a rear end side surface of the layered substrate 37 and covers the laser element 32, the laser driver circuit 33, the ground potential pad 43, and the like.

A configuration example of the first cap member 50 will be described with reference to Fig. 6. Fig. 6 is a displacement cross-sectional view of the first cap member 50 taken along line VI-VI in Fig. 4. The first cap member 50 includes a first through hole 51, a recess 52, and a second through hole 53.

The first through hole 51 penetrates the first cap member 50 in the axial direction and is connected to the recess 52. The first through hole 51 accommodates at least a part of the optical fiber 34. Furthermore, the first through hole 51 may accommodate at least a part of the coil 38 as illustrated in the drawing. As in the present embodiment, in a case where the optical transmitter module 30 includes a plurality of the optical fibers 34, a single first through hole 51 may accommodate the plurality of optical fibers 34, or a plurality of first through holes 51 may be provided and each of them may accommodate separated one optical fiber 34.

A diameter of the optical fiber 34 may change along the axial direction. In the example of Fig. 6, the optical fiber 34 has a small diameter portion where a core 34a is exposed, and a large diameter portion where the core 34a is covered with a clad portion (not illustrated in Fig. 6) and a covering portion 34c. In such a case, the first through hole 51 may have a stepped hole shape as illustrated, that is, the diameter may change along the axial direction depending on a change in the diameter of the optical fiber 34.

The recess 52 is formed so as to open toward a surface (distal end side) facing the layered substrate 37 in the first cap member 50, and accommodates the laser driver circuit 33. In the example of Fig. 6, the recess 52 further accommodates the laser element 32 and the wire 39. The second through hole 53 penetrates the first cap member 50 in the axial direction and accommodates at least a part of the heat dissipation wiring 35.

By providing the first through hole 51, the recess 52, and the second through hole 53 in the first cap member 50, positioning of each accommodation target (optical fiber 34, laser element 32, laser driver circuit 33, heat dissipation wiring 35, and the like) is facilitated, which improves manufacturability. In particular, when the first through hole 51 is configured to have a stepped hole shape as illustrated in Fig. 6, positioning of the optical fiber 34 is further facilitated.

In the present embodiment, the coil 38 covers the outer periphery of the boundary between a portion of the optical fiber 34 accommodated by the first cap member 50 and a portion of the optical fiber 34 not accommodated by the first cap member 50. In this way, it is possible to prevent the optical fiber 34 from being damaged at the boundary of the first cap member 50.

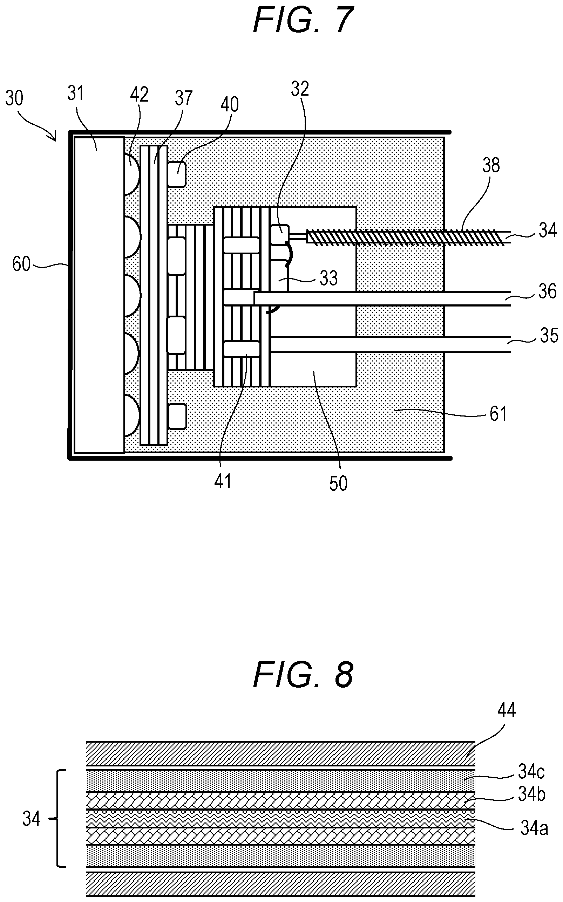

The optical transmitter module 30 is sealed with a molding agent. Fig.7 illustrates an example of sealing with the molding agent 61.

A second cap member 60 having a tubular shape is attached to the optical transmitter module 30. The second cap member 60 has, for example, a bottomed tubular shape, but may have a tubular shape penetrating without a bottom. The second cap member 60 is made of, for example, resin but may be made of any material. The second cap member 60 is disposed so as to cover, from the distal end side of the optical transmitter module 30, the entire image sensor 31, the layered substrate 37, and the first cap member 50, and the inside of the second cap member 60 is filled with the molding agent 61. As a result, the components are more reliably fixed to the layered substrate 37. Note that it does not matter if the molding agent 61 is filled into the first cap member 50 (that is, the first through hole 51, the recess 52, and the second through hole 53) or not.

In particular, the molding agent 61 fixes at least a part of the optical fiber 34. As described above, the optical fiber 34 can be prevented from coming off from the laser element 32 by the sealed structure using the molding agent 61.

The whole including the second cap member 60 and the molding agent 61 can be disposed inside the hard portion 11a that is not deformed.

In the present embodiment, the coil 38 covers the outer periphery of the boundary between a portion of the optical fiber 34 fixed with the molding agent 61 and a portion of the optical fiber 34 not fixed with the molding agent 61. In this way, it is possible to prevent the optical fiber 34 from being damaged at the boundary of the molding agent 61.

In a portion not fixed with the molding agent 61, the optical fiber 34 is rotatable and axially movable with respect to other structures inside the endoscope 11 via the coil 38. This reduces friction between the optical fiber 34 and other structures when the endoscope 11 is bent, and allows the motion (for example, bending motion or straight motion) of the endoscope 11 to be smoothly performed.

### Second Embodiment

In the second embodiment, the protective member of the first embodiment is changed from the coil 38 to a tube. Hereinafter, description of parts common to the first embodiment may be omitted.

Fig. 8 illustrates a configuration example of an optical fiber 34 and a tube 44 according to the second embodiment. At least a part of the outer periphery of the optical fiber 34 is covered with the tube 44. The axial range of a portion of the optical fiber 34 covered with the tube 44 can be similar to the axial range of a portion of the optical fiber 34 covered with the coil 38 in the first embodiment.

The optical fiber 34 includes a core 34a, a cladding portion 34b, and a covering portion 34c. The tube 44 is a member different from the covering portion 34c of the optical fiber 34, and is disposed further on the outer peripheral side of the covering portion 34c.

Although a material or the like of the tube 44 can be arbitrarily designed, a material and a structure having sufficient strength to prevent breakage of the optical fiber 34 are preferable. The tube 44 may be made of, for example, resin.

Also in the second embodiment, since the optical fiber 34 is covered with the tube 44, it is possible, when the endoscope performs the bending operation, to reduce the possibility that the optical fiber is damaged (for example, it is crushed or folded) at least in the covered portion. For example, the tube 44 can relieve a radial pressure applied to the optical fiber 34 in the endoscope 11, which can prevent the optical fiber 34 from being crushed.

Furthermore, in a portion not fixed with the molding agent 61, the optical fiber 34 is rotatable and axially movable with respect to other structures inside the endoscope 11 via the tube 44. This reduces friction between the optical fiber 34 and other structures when the endoscope 11 is bent, and allows the motion (for example, bending motion or straight motion) of the endoscope 11 to be smoothly performed.

### Other Embodiments

The first and second embodiments may be combined. That is, the optical fiber 34 may be covered with the tube 44 and the coil 38 may be wound around the outer periphery of the tube 44, or the coil 38 may be wound around the optical fiber 34 and the outer periphery of the coil 38 may be covered with the tube 44.

The first cap member 50 may be omitted. The second cap member 60 and the molding agent 61 may be omitted. Even in these cases, the breakage of the optical fiber 34 can be prevented by the coil 38 or the tube 44.

The protective member may have a structure other than that of the coil 38 or the tube 44, and may have, for example, a mesh shape.

The endoscope system 1 or the endoscope 11 is not limited to the configurations and functions described in the present description and may have configurations and functions of known endoscopes.

The present disclosure includes the following specified matters.

### [Specified matter 1]

An optical transmitter module according to the present invention including:
an image sensor, a laser element, a laser driver circuit, an optical fiber, and a layered substrate, wherein
the optical fiber is connected to the laser element, and
at least a part of an outer periphery of the optical fiber is covered with a protective member.

### [Specific matter 2]

The optical transmitter module according to specified matter 1, wherein
the optical transmitter module includes a cap member made of glass,
the cap member has a through hole that accommodates at least a part of the optical fiber, and
the protective member covers an outer periphery of a boundary between a portion of the optical fiber accommodated by the cap member and a portion of the optical fiber not accommodated by the cap member.

### [Specified matter 3]

The optical transmitter module according to specified matter 1 or 2, wherein
at least a part of the optical fiber is fixed with a molding agent, and
the protective member covers an outer periphery of a boundary between a portion of the optical fiber fixed with the molding agent and a portion of the optical fiber not fixed with the molding agent.

### [Specified matter 4]

The optical transmitter module according to any one of specified matters 1 to 3, wherein
the optical transmitter module includes a plurality of the optical fibers, and
the single protective member covers an outer periphery of the plurality of optical fibers.

### [Specified matter 5]

The optical transmitter module according to any one of specified matters 1 to 4, wherein the protective member is a coil.

### [Specified matter 6]

The optical transmitter module according to any one of specified matters 1 to 4, wherein the protective member is a tube and is a member different from a covering portion of the optical fiber.

### [Specified matter 7]

An endoscope including the optical transmitter module according to any one of specified matters 1 to 6.

### [Specified matter 8]

The endoscope according to specified matter 7, wherein the protective member covers an outer periphery of the optical fiber at least over an entire length of a bend portion of the endoscope.

### Reference Signs List

- 1: Endoscope system
- 10: Endoscope unit
- 11: Endoscope
- 11a: Hard portion
- 11b: Bend portion
- 11c: Flexible portion
- 12: Operation unit
- 13: Connector unit
- 14: Light irradiation means
- 15: Rod
- 20: Processor
- 21: Connector unit
- 30: Optical transmitter module
- 31: Image sensor
- 32: Laser element
- 33: Laser driver circuit
- 34: Optical fiber
- 34a: Core
- 34b: Cladding portion
- 34c: Covering portion
- 35: Heat dissipation wiring
- 36: Electric signal wiring
- 37: Layered substrate
- 38: Coil (protective member)
- 39: Wire
- 40: Passive component
- 41: Cable wiring pad
- 43: Ground potential pad
- 44: Tube (protective member)
- 50: First cap member (cap member)
- 51: First through hole (through hole)
- 52: Recess
- 53: Second through hole
- 60: Second cap member
- 61: Molding agent

All publications, patents, and patent applications cited herein are hereby incorporated by reference in their entirety.

## Claims

1. An optical transmitter module comprising an image sensor, a laser element, a laser driver circuit, an optical fiber, and a layered substrate, wherein
the optical fiber is connected to the laser element, and
at least a part of an outer periphery of the optical fiber is covered with a protective member.

2. The optical transmitter module according to claim 1, wherein
the optical transmitter module comprises a cap member made of glass,
the cap member has a through hole that accommodates at least a part of the optical fiber, and
the protective member covers an outer periphery of a boundary between a portion of the optical fiber accommodated by the cap member and a portion of the optical fiber not accommodated by the cap member.

3. The optical transmitter module according to claim 1 or 2, wherein
at least a part of the optical fiber is fixed with a molding agent, and
the protective member covers an outer periphery of a boundary between a portion of the optical fiber fixed with the molding agent and a portion of the optical fiber not fixed with the molding agent.

4. The optical transmitter module according to any one of claims 1 to 3, wherein
the optical transmitter module comprises a plurality of the optical fibers, and
the single protective member covers an outer periphery of the plurality of optical fibers.

5. The optical transmitter module according to any one of claims 1 to 4, wherein the protective member is a coil.

6. The optical transmitter module according to any one of claims 1 to 4, wherein the protective member is a tube and is a member different from a covering portion of the optical fiber.

7. An endoscope including the optical transmitter module according to any one of claims 1 to 6.

8. The endoscope according to claim 7, wherein the protective member covers an outer periphery of the optical fiber at least over an entire length of a bend portion of the endoscope.
